# EUROPEAN PATENT APPLICATION

(11) **EP 3 435 379 A1**
(43) Date of publication of application: **30.01.2019**
(21) Application number: 17183458.3
(22) Date of filing: 27.07.2017
(51) Int. Cl.: G16H 10/40

(54) **AUGMENTING MEASUREMENT VALUES OF BIOLOGICAL SAMPLES**

(71) Applicant: Roche Diagnostics GmbH, 68305 Mannheim (DE); F. Hoffmann-La Roche AG, 4070 Basel (CH)
(72) Inventor: RODA JIMÉNEZ, Javier, 6343 Rotkreuz (CH); ZHANG, Hao, 6343 Rotkreuz (CH); REIMERS, Jens Jacob, 6343 Rotkreuz (CH)
(74) Representative: Gyenge, Zoltán

(57) **Abstract**

Disclosed is a method and system to carry out the method comprising the steps: analyzing biological samples of a patient to obtain measurement values (A) and (B) according to a test order; a laboratory middleware (30) receiving the measurement values (A) and (B); providing a virtual analyzer (50) comprising a workflow engine (52) and an algorithm calculator (54)- distinct from the laboratory middleware (30); the workflow engine (52) receiving input data (I) comprising measurement values (A) and (B) and second input data (II) comprising patient data from the laboratory middleware (30); the algorithm calculator (54) processing the input data (I) and (II) using the clinical algorithm (80) as instructed by the workflow engine (52) and transmitting interpretation support data (O) to the workflow engine (52); and the laboratory middleware (30) augmenting the measurement values (A) and (B) with the interpretation support data (O) received from the virtual analyzer (50).

## Description

### TECHNICAL FIELD

The application relates to a computer implemented method for augmenting measurement values of biological samples - obtained by analysis using laboratory instruments - to provide interpretation support, in particular clinical interpretation support for physicians.

The application further relates to a laboratory system for processing biological samples configured for augmenting measurement values to provide interpretation support.

The application further relates to a web-based system configured for augmenting measurement values obtained by analysis of biological samples by laboratory system(s).

### BACKGROUND

In analytical laboratories, in particular clinical laboratories, a multitude of analyses on biological samples are executed by analytical systems in order to determine the physiological state of patients.

The creation of high medical value products is of great importance for patients and medical professionals alike. Multiple data sets from clinical laboratories and other information systems, when combined, have the potential to generate medical and clinical insights providing an added medical value beyond that of these data sets in isolation. In order to provide medical professionals support for interpretation of the results of analyses of biological samples, the measurement values - obtained from laboratory systems by analysis of biological samples - are augmented with so-called interpretation support data, obtained commonly by the use of medical algorithms. Currently, interpretation support data by medical algorithms is often generated through external platforms or even by applying such medical algorithms manually. However, transferring data between various systems is disadvantageous, as data integrity cannot be guaranteed and there is a high risk due to e.g. human error in case this data transfer takes place manually. Furthermore, the use of external platforms to apply medical algorithms has the additional disadvantage that it is difficult to validate the correctness of the end result. On one hand, already the selection of the external platform for each medical algorithm might not be consistent amongst medical professionals. Also, medical professionals might not be aware of the newest developments in medical algorithms, in the identification of new correlations between biomarkers, which when combined by a medical algorithm in view of patient data have the potential to provide great added medical value. Thus there is a risk that using currently known external platforms, medical professionals rely on interpretation support provided by outdated and/or incomplete and/or potentially even erroneous medical algorithms or inconsistent input data.

On the other hand, the validity of the medical algorithm and its output is questionable if the medical algorithm or the platform hosting it lies outside of the control of the provider of the measurement values (or the instruments and/or assays generating them) on which the calculations will be based.

In order to address such issues, laboratory systems for augmenting measurement results of samples are known which hardcode/ incorporate medical algorithms into the laboratory IT systems, in particular into the laboratory middleware. However, such systems which hardcode/ incorporate medical algorithms lack modularity and flexibility, wherein the provision of a new medical algorithm or updating an existing one has an impact on the entire laboratory IT system. Furthermore, as soon as any system generates/ changes or in any way affects a measurement value or medical result that system is considered a medical device and falls under stringent regulatory approvals. Hence any software application or algorithm associated with interpretation support data (a medical claim e.g. risk factor calculation, treatment suggestion, or clinical decision support systems) require stringent regulatory requirements and imply complex changes to products. This has the consequence that known IT systems with interpretation support hardcoded into laboratory middleware suffer from the drawback that each time a new medical algorithm is to be provided or an existing one needs to be updated, the entire IT systems needs to be re-validated and approved by regulatory bodies - a costly and time consuming process.

There is therefore a need for a method - respectively a laboratory system - for augmenting measurement values of biological samples by laboratory instruments to provide interpretation support which overcomes both the disadvantages of using external platforms for medical algorithms but also the drawbacks faced by known laboratory IT solutions with interpretation support hardcoded/ incorporated into the laboratory middleware.

In other words there is a need for a method - respectively a laboratory system - for augmenting measurement values of biological samples by laboratory instruments which provides guaranteed interpretation support relying on validated, up-to date and complete medical algorithms on one hand and provides flexibility and modularity on the other hand.

### SUMMARY

Disclosed herein is a method comprising the steps:
- receiving a test order corresponding to a patient by a laboratory middleware;
- one or more laboratory instruments analyzing one or more biological samples of the patient to obtain a first measurement value and a second measurement value therefrom, in response to an instruction from the laboratory middleware according to the test order;
- the laboratory middleware receiving the first measurement value and the second measurement value obtained by analysis of the one or more biological samples of the patient;
- providing a virtual analyzer comprising a workflow engine and an algorithm calculator, the virtual analyzer being distinct from the laboratory middleware;
- the workflow engine receiving a first input data comprising the first measurement value and the second measurement value from the laboratory middleware;
- the workflow engine receiving from the laboratory middleware a second input data comprising patient data corresponding to the patient;
- providing a clinical algorithm to the algorithm calculator, the clinical algorithm defining processing steps to apply on the first input data and second input data;
- the workflow engine instructing the algorithm calculator to apply the clinical algorithm on the first input data and second input data in response to the test order received from the laboratory middleware;
- the algorithm calculator processing the first input data and second input data using the clinical algorithm and transmit interpretation support data to the workflow engine;
- the workflow engine forwarding the interpretation support data to the laboratory middleware;
- the laboratory middleware augmenting the first measurement value and the second measurement value with the interpretation support data received from the virtual analyzer.

Further disclosed herein is a laboratory system for processing biological samples comprising:
- a plurality of laboratory instruments for analyzing biological samples, each laboratory instrument providing a measurement value obtained by analysis of a biological sample;
- a laboratory middleware communicatively connected to the plurality of laboratory instruments and
- an algorithm calculator comprising a clinical algorithm,
wherein the laboratory system is configured to perform the steps according to embodiments of the method disclosed.

Further disclosed is a web-based system comprising:
- a user interface for receiving first input data indicative of the first measurement value and the second measurement value obtained by analysis of the one or more biological samples of the patient and for receiving second input data comprising patient data corresponding to the patient;
- a virtual analyzer comprising a workflow engine and an algorithm calculator comprising a clinical algorithm;
wherein the web-based system is configured to perform the steps according to embodiments of the method disclosed.

Further disclosed herein is a computer-readable medium storing instructions thereon which when executed by a computer system controls the computer system to perform the steps of any one of the embodiments of the method disclosed.

Embodiments of the disclosed method/ system are particularly advantageous as they fulfil two seemingly contradictory requirements, namely eliminating the disadvantages of external platforms hosting the medical algorithms on one hand and also avoiding the lack of flexibility of laboratory systems with interpretation support hardcoded/ incorporated into laboratory middleware.

In other words, embodiments of the disclosed method/ system are particularly advantageous as they ensure both the quality of the interpretation support data and also provide flexibility by avoiding the need for re-validation of the entire lab IT solution each time a medical algorithm is updated or a new one is added.

Embodiments of the disclosed method/ system achieve the first goal - validated, up-to-date and complete medical algorithms - by way of a system-internal algorithm calculator applying validated clinical algorithms.

With respect to the second main goal - flexibility by avoidance of repeated regulatory approval of each new/ updated medical algorithm - embodiments of the disclosed method/ system achieve this goal by "isolating" / "encapsulating" the medical algorithms into a virtual analyzer, which comprises a workflow engine to control an algorithm calculator to apply/ execute the clinical algorithm. The workflow engine of the virtual analyzer has the further task of ensure quality and availability of input data and output data.

In this way a clear separation is provided between the individual algorithms, the algorithm calculator and the laboratory middleware. Hence, any change or addition of a new medical algorithm limits the need for both technical and regulatory (re)validation to the particular medical algorithms. As a final result, the disclosed method/ system ensures that by using validated medical algorithms and executing them on a system-internal algorithm calculator based on internally-generated and processed measurement values, the interpretation support data provided is correct, complete and validated (both technically as well as regulatory).

As a final conclusion, embodiments of the disclosed method/ system provide the benefits of combining multiple measurement results of biological samples and patient data to produce interpretation support data for medical professionals in a flexible, safe and reliable environment.

### BRIEF DESCRIPTION OF THE DRAWINGS

Further characteristics and advantages of the disclosed method / device /system will in the following be described in detail by means of the description and by making reference to the following drawings:
- Fig. 1: a highly schematic block diagram of an embodiment of the disclosed laboratory system;
- Fig. 2A: a highly schematic block diagram of a further embodiment of the disclosed laboratory system, wherein the virtual analyzer is deployed in a cloud environment;
- Fig. 2B: a highly schematic block diagram of a web-based system comprising a virtual analyzer deployed in a cloud environment;
- Fig. 3: a swim-lane diagram depicting an embodiment of the disclosed method; and
- Fig. 4: a swim-lane diagram depicting a further embodiment of the disclosed method showing improvement of the clinical algorithm(s) based on feedback indicative of interpretation support accuracy.

Note: The figures are not drawn to scale, are provided as illustration only and serve only for better understanding but not for defining the claimed scope. No limitations of any features should be inferred from these figures.

### DETAILED DESCRIPTION

Certain terms will be used in this patent application, the formulation of which should not be interpreted to be limited by the specific term chosen, but as to relate to the general concept behind the specific term.

The term **'laboratory instrument'** as used herein encompasses any apparatus or apparatus component operable to execute one or more processing steps / workflow steps on one or more biological samples. The expression 'processing steps' thereby refers to physically executed processing steps such as centrifugation, aliquotation, sample analysis and the like. The term 'laboratory instrument' covers pre-analytical instruments, post-analytical instruments and also analytical instruments.

The term **'pre-analytical instrument'** as used herein comprises one or more lab-devices for executing one or more pre-analytical processing steps on one or more biological samples, thereby preparing the samples for one or more succeeding analytical tests. A pre-analytical processing step can be, for example, a centrifugation step, a capping-, decapping- or recapping step, an aliquotation step, a step of adding buffers to a sample and the like. The expression **'analytical system'** as used herein encompasses any monolithic or multi-modular laboratory device comprising one or more lab-devices or operative units which are operable to execute an analytical test on one or more biological samples.

The term **'post-analytical instrument'** as used herein encompasses any laboratory instrument being operable to automatically process and/or store one or more biological samples. Post-analytical processing steps may comprise a recapping step, a step for unloading a sample from an analytical system or a step for transporting said sample to a storage unit or to a unit for collecting biological waste.

The term **'analyzer' / 'analytical instrument'** as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such an analyzer are clinical chemistry analyzers, coagulation chemistry analyzers, immunochemistry analyzers, urine analyzers, nucleic acid analyzers, tissue analyzers (incl. morphological stainers and histochemical stainers) used to detect the result of chemical or biological reactions or to monitor the progress of chemical or biological reactions.

The term **'laboratory system'** as used herein encompasses any system for the use in a laboratory comprising plurality of laboratory instruments operatively connected to a control unit.

The term **'test order'** as used herein refers to any data object, computer loadable data structure, modulated data representing such data being indicative of one or more laboratory processing steps to be executed on a particular biological sample. For example, a test order record may be a file or an entry in a database. According to embodiments disclosed herein, a test order can indicate a test order for an analytical test if, for example, the test order comprises or is stored in association with an identifier of an analytical test to be executed on a particular sample. Alternatively or additionally, the test order may refer to pre- and/or post-analytical processing steps to be performed on the biological sample.

The term **'laboratory middleware'** as used herein encompasses any physical or virtual processing device configurable to control a laboratory system comprising a plurality of laboratory instruments in a way that workflow(s) and workflow step(s) are conducted by the laboratory system. The laboratory middleware may, for example, instruct the laboratory system (or a specific instrument thereof) to conduct pre-analytical, post analytical and analytical workflow(s)/ workflow step(s). The laboratory middleware may receive information from a data management unit regarding which steps need to be performed with a certain sample, in particular in the form of a test order indicative of processing steps (such as analytical measurements) to be carried out on a biological sample. According to various embodiments, the laboratory middleware may comprise or be connected to a user interface for inputting of test orders corresponding to the biological samples. Alternatively or additionally, the laboratory middleware comprises a communication interface configured to receive the test orders corresponding to the biological samples.

In some embodiments, the laboratory middleware might be integral with a data management unit, may be comprised by a server computer and/or be part of one instrument or even distributed across multiple instruments of the laboratory system. The laboratory middleware may, for instance, be embodied as a programmable logic controller running a computer-readable program provided with instructions to perform operations.

The term **'virtual analyzer'** as used herein encompasses any virtual or physical processing device storing instructions which when executed cause the processing of input data to produce output data by applying computational steps according to a clinical algorithm.

The term **'communication network'** as used herein encompasses any type of wireless network, such as a WIFI, GSM, UMTS or wired network, such as Ethernet or the like. In particular, the communication network can implement the Internet protocol IP. For example, the communication network comprises a combination of cable-based and wireless networks. In embodiments wherein units of the system are comprised within one laboratory instrument, the communication network comprises communication channels within an instrument.

The term **'user interface'** as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

The term **'workflow'** as used herein refers to a collection of workflow steps /processing steps. According to particular embodiments, the workflow defines a sequence in which the processing steps are carried out.

The term **'workflow step'** or **'processing step'** as used herein encompasses any activity belonging to a workflow. The activity can be of an elementary or complex nature and is typically performed at or by one or more instrument(s).

The terms **'sample', 'patient sample'** and **'biological sample'** refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The terms **'aliquot', 'patient sample aliquot'** and **'biological sample aliquot'** refer to a portion of the sample, patient sample or biological sample usually obtained by aliquoting, i.e. dividing the biological sample, in particular using a pipetting process. In this context the biological sample is referred to as primary sample and the tube in which it resides is referred to as primary sample tube while the sample portions divided from the primary sample are called aliquots and the tube(s) in which they reside are referred to as aliquot tubes or secondary tubes. An aliquot(s) of a biological sample is usually created into a secondary sample tube or sample plate well separate from the primary sample tube or sample plate well.

The term **'analyte'** as used herein refers to a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term **'analysis'** or '**analytical test'** as used herein encompasses a laboratory procedure characterizing a parameter of a biological sample, e.g. light absorption, fluorescence, electrical potential or other physical or chemical characteristics of the reaction to provide the measurement data.

The term **'analytical data'** as used herein encompasses any data that is descriptive of a result of a measurement of a biological sample. In case of a calibration the analytical data comprises the calibration result, i.e. calibration data. In particular, the analytical data comprises an identifier of the sample for which the analysis has been performed and data being descriptive of a result of the analysis,

Particular embodiments of the disclosed method/ system shall be described as follows with reference to the figures.

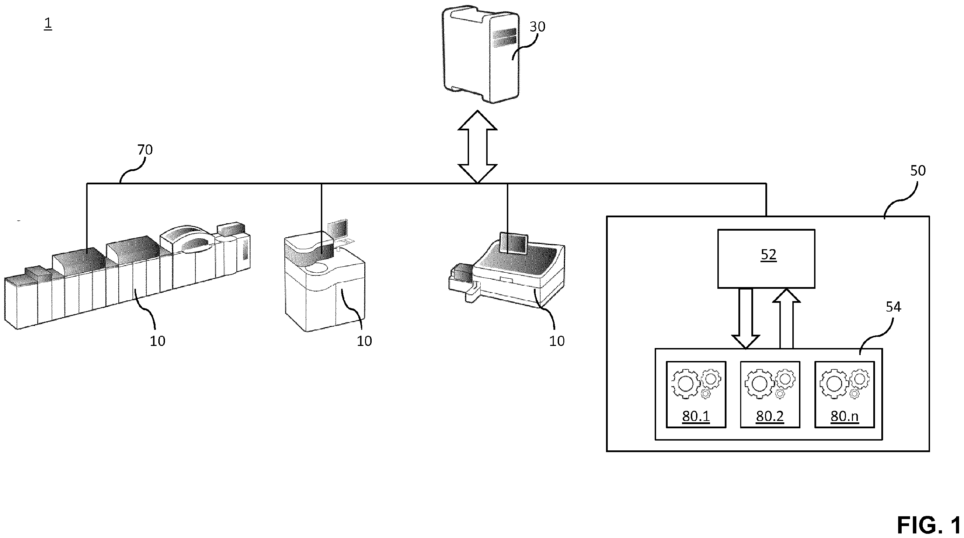

Figure 1 shows a highly schematic block diagram of an embodiment of the disclosed laboratory system 1. As illustrated, the laboratory system 1 comprises a laboratory middleware 30, one or more laboratory instruments 10 and a virtual analyzer 50 connected with each other by a communication network 70.

The virtual analyzer 50 comprises a workflow engine 52 and an algorithm calculator 54. The workflow engine 52 is configured to collect first input data I comprising a measurement values obtained by analysis of biological sample(s) of the patient as well as second input data II comprising patient data corresponding to the patient. The workflow engine 52 is hence responsible for checking quality/format of input data.

According to embodiments disclosed herein, the laboratory middleware 30 is configured to retrieve the patient data from a host system 40 such as a hospital information system HIS, an electronic medical record EMR and/or a computerized Physician Order Entry CPOE.

The patient data comprises one or more from the non-exhaustive list comprising:
- age of a patient;
- life stage of a patient, (e.g. pre- or post-menopausal);
- one or more medical condition(s) of a patient;
- physiological characteristics of a patient, such as weight, height, gender, etc.
- a list of past and/or current drug(s) administered to the patient and respective dosage(s);
- past and/or current treatments of the patient;
- patient demographics.

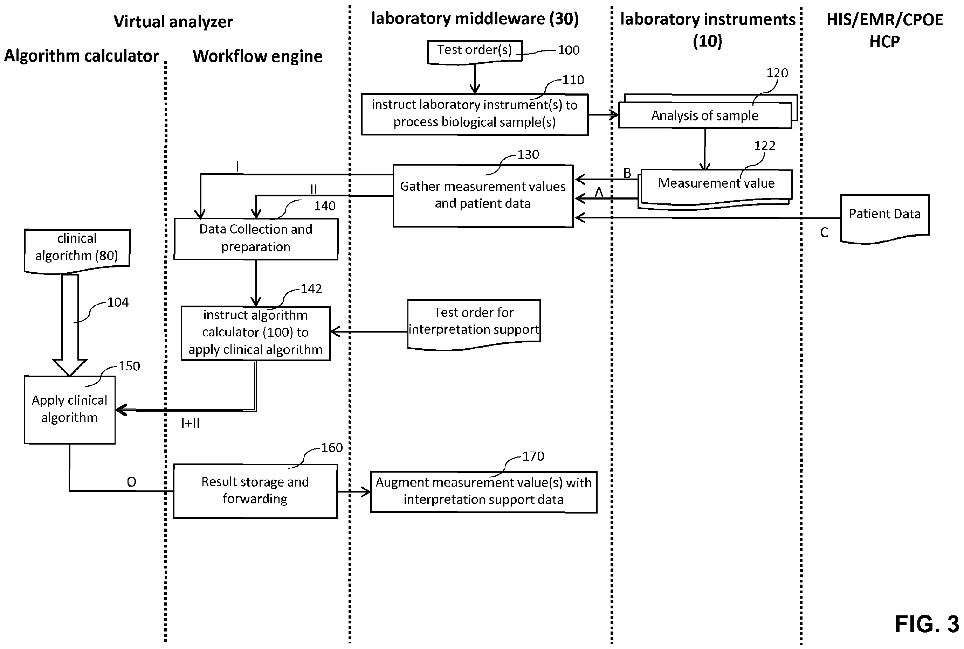

The algorithm calculator 54 is configured to apply clinical algorithm(s) 80 on the first input data I respectively second input data II so as to thereby generate interpretation support data O. The clinical algorithms 80.1, 80.2-80.n are provided within the algorithm calculator 54 in a plugin-type fashion, each clinical algorithm 80.1, 80.2-80.n being self-contained in that defines all parameters and processing steps to be applied on the on the first input data I respectively second input data II to generate the corresponding interpretation support data O (see figures 3 through 5 and related description for first input data I, second input data II and interpretation support data O). In other words, each clinical algorithm 80.1, 80.2-80.n is by itself deterministic, in that the interpretation support data O is determined purely based on the first input data I respectively second input data II and the clinical algorithm 80.

According to particular embodiments disclosed herein the clinical algorithm 80 is provided as of software package defining processing and calculation steps to be applied on the input data I and II as well as various parameters, value ranges, etc. Alternatively or additionally, the clinical algorithm 80 is defined as a mathematical formula.

According to particular embodiments disclosed herein, each clinical algorithm 80 is regulatory approved and validated by itself in order to guarantee the interpretation support data O generated. As such, the clinical algorithms 80 are deployed on the virtual analyzer 50 analogous to the way analytical tests (also known as assays) are deployed on laboratory instruments 10. Following the analogy with physical laboratory instruments 10 which produce measurement values by analyzing biological samples using an assay(s), the virtual analyzer 50 produces interpretation support data O by processing (analyzing) other measurement values (first input data I) in view of patient data (second input data II) using clinical algorithm(s) 80. Also analogous with physical laboratory instruments 10, the assays of which are by themselves validated and regulatory approved, the clinical algorithm(s) 80 deployed on the algorithm calculator 54 of the virtual analyzer 50 are validated and regulatory approved as well.

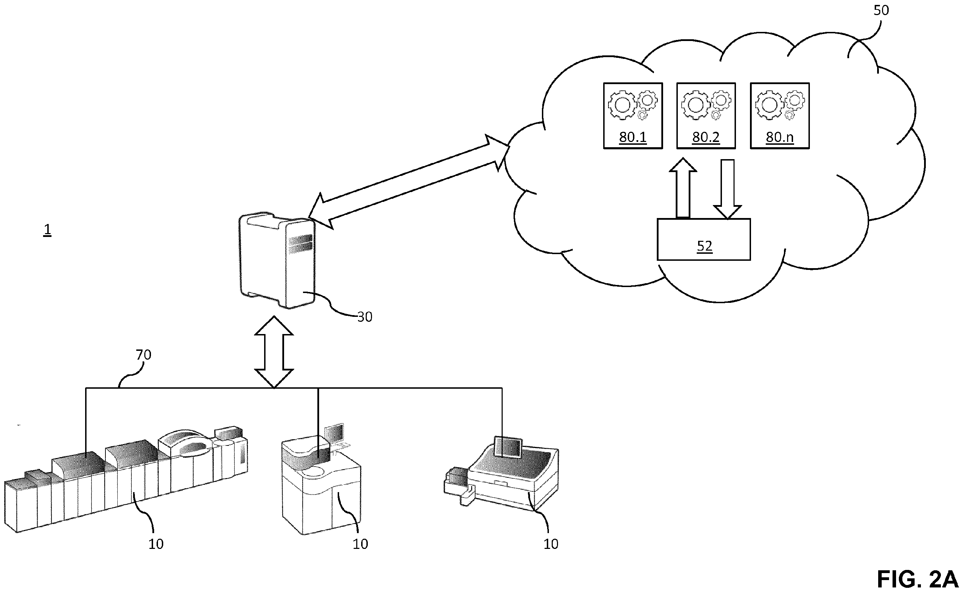

Figure 2A shows a highly schematic block diagram of a further embodiment of the disclosed laboratory system 1, wherein the virtual analyzer 50 is deployed on a remote server, in particular a cloud environment.

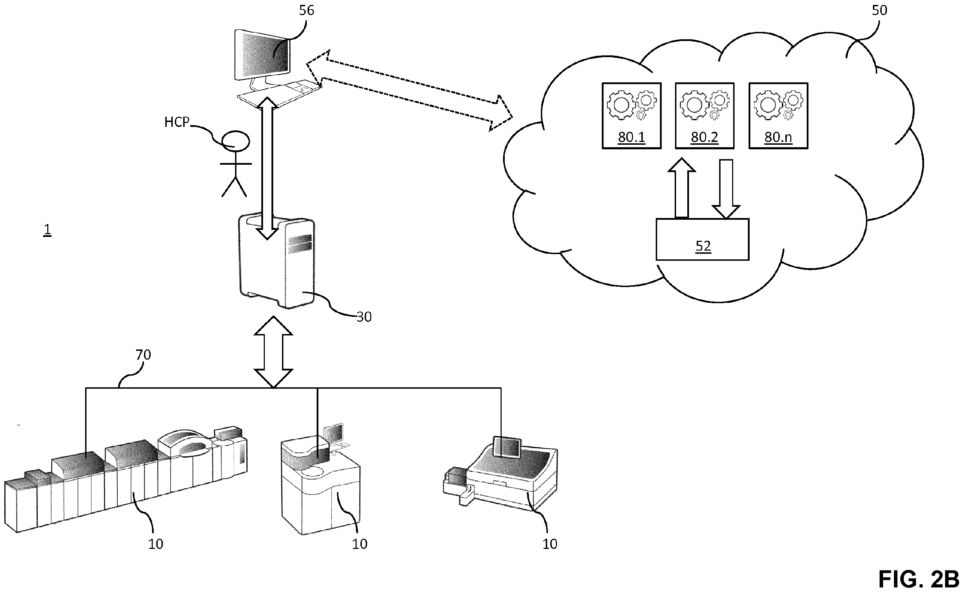

Figure 2B shows a web-based system comprising a user interface 56 for receiving first input data I indicative of the first measurement value A and the second measurement value B obtained by analysis of the one or more biological samples of the patient and for receiving second input data II comprising patient data corresponding to the patient. As in the embodiment shown on figure 2A, the virtual analyzer 50 comprising a workflow engine 52 and an algorithm calculator 54 comprising one or more clinical algorithm(s) 80 is deployed on a remote server, in particular a cloud environment.

Figure 3 shows a swim-lane diagram depicting an embodiment of the disclosed method. In a preparatory step, a virtual analyzer 50 comprising a workflow engine 52 and an algorithm calculator 54 is provided, the virtual analyzer 50 being distinct from the laboratory middleware 30. The fact that the virtual analyzer 50 is distinct (physically and/or logically) from the laboratory middleware 30 is important as it ensures that any change of the virtual analyzer 50, or of its workflow engine 52, algorithm calculator 54 (or the clinical algorithm 80 deployed on the latter) does not necessitate a (re)-validation and/or regulatory (re)-approval of the laboratory middleware 30. The term distinct, with reference to the virtual analyzer 50 being distinct (physically and/or logically) from the laboratory middleware 30 - refers herein to a logical and/or physical separation of the two, ensuring that no change of the virtual analyzer 50 transpires to the laboratory middleware 30 and the other way around.

As a further preparatory step 104, one or more clinical algorithm(s) 80 are provided to the algorithm calculator 54. As mentioned above, the clinical algorithm(s) 80 are installed/ deployed onto the algorithm calculator 54 as pre-validated and regulatory approved calculation packages with well-defined inputs (first and second input data I and II) and deterministic output (interpretation support data O). Hence the clinical algorithm 80 defines all processing steps to be applied on the first input data I and second input data II. According to embodiments of the disclosed method/ system, the one or more clinical algorithm(s) 80 are installed on the virtual analyzer 50 from a clinical algorithm catalogue, the clinical algorithm catalogue being a repository of validated and regulatory approved clinical algorithms. In particular, the clinical algorithm catalogue is stored on a remote server maintained by a provider with oversight from a regulatory body.

As it can be seen in this figure, the method is triggered by the receipt of a test order corresponding to a patient by the laboratory middleware 30 in a step 100. According to various embodiments of the disclosed method, the test order is either recorded directly on a user interface of the laboratory middleware 30 or received from a host system - such as a laboratory information system LIS.

Thereafter, in a step 110, the laboratory middleware 30 instructs one or more laboratory instruments 10 - according to the test order - to analyze one or more biological samples of the patient.

Once the one or more biological samples of the patient are available (i.e. collected from the patient and entered the laboratory), in a step 120, the one or more laboratory instruments 10 analyze one or more biological samples of the patient to obtain - in a step 122 - a first measurement value A respectively a second measurement value B therefrom.

In a step 130, the laboratory middleware 30 receives the first measurement value A and the second measurement value B obtained by analysis of the one or more biological samples of the patient.

In a subsequent step 140, the workflow engine 52 collects the first input data I comprising the first measurement value A and the second measurement value B from the laboratory middleware 30 and second input data II comprising patient data corresponding to the patient. According to embodiments of the disclosed method, also part of step 140 is the preparation of the collected data by the workflow engine 52 in order to ensure that the input data is in a format as expected by the algorithm calculator 54. For example, the workflow engine 52 of the virtual analyzer 50 is configured to translate the first measurement value A and the second measurement value B received in HL7 format (common for laboratory middleware and laboratory information systems) into an XML format expected from the algorithm calculator 54.

According to embodiments of the disclosed method, as part of data preparation, the workflow engine 52 of the virtual analyzer 50 is further configured to validate the first and second input data I, II based on defined validation criteria (technical and or medical validation).

According to further embodiments of the disclosed method, as part of data preparation, the workflow engine 52 of the virtual analyzer 50 is further configured to trigger the analysis of the one or more biological samples by the one or more laboratory instruments 10 to obtain a first measurement value A respectively a second measurement value B. The workflow engine 52 of the virtual analyzer 50 does this knowing the first input data I (comprising the first measurement value A and the second measurement value B) required for obtaining the requested interpretation support data O.

In a following step 142, the workflow engine 52 of the virtual analyzer 50 instructs the algorithm calculator 54 to apply the clinical algorithm 80 on the first input data I and second input data II in response to the test order received from the laboratory middleware 30. According to embodiments of the disclosed method, in step 142 the workflow engine 52 also determines the particular clinical algorithm 80 to process the first input data I and second input data II corresponding to the test order received from the laboratory middleware 30.

In response to the instructions from the workflow engine 52, in a step 150, the algorithm calculator 54 processes the first input data I and second input data II using the corresponding clinical algorithm 80 and transmits the resulting interpretation support data O to the workflow engine 52.

In a subsequent step 160, the workflow engine 52 forwards the interpretation support data O to the laboratory middleware 30.

According to embodiments of the disclosed method/ system, as part of step 160, the workflow engine 52 validates the interpretation support data O received from the algorithm calculator 54 before forwarding to the to the laboratory middleware 30.

According to further embodiments of the disclosed method/ system, as part of step 160, the workflow engine 52 formats the interpretation support data O received from the algorithm calculator 54 into a format expected/ accepted by the laboratory middleware 30. For example, the workflow engine 52 of the virtual analyzer 50 is configured to translate the interpretation support data O received in an XML format from the algorithm calculator 54 into an HL7 format (common for laboratory middleware and laboratory information systems) accepted by the laboratory middleware 30.

In a subsequent step 170, the laboratory middleware 30 augments the first measurement value A and the second measurement value B with the interpretation support data O received from the virtual analyzer 50. According to further embodiments of the disclosed method/ system, the interpretation support data O is made available to the physician, health care professional HCP respectively lab operator directly via the laboratory middleware 30 and/or a dedicated user interface - such as a web portal. In the context of the present disclosure, the term augmenting refers to presenting the measurement values obtained by analysis of the biological sample alongside/ in the context of the interpretation support data O calculated based on the same measurement values in combination with patient data.

According to embodiments herein disclosed, processing of the first input data I and second input data II using the clinical algorithm 80 comprises determination of a risk score indicative of the patient to have and/or to acquire a certain medical condition, the interpretation support data O comprising said risk score.

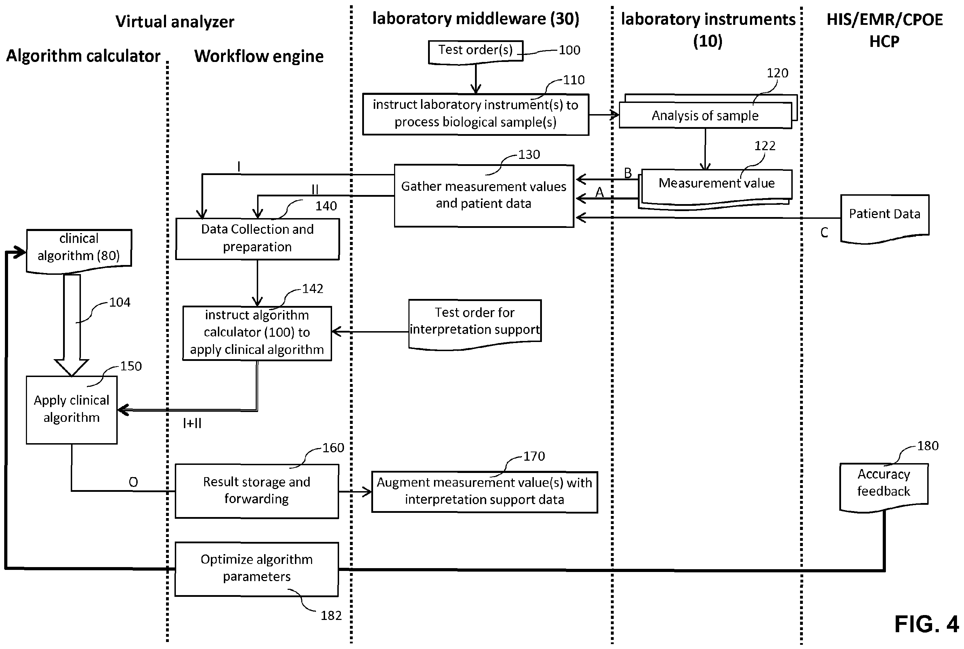

Turning now to figure 4, showing a swim-lane diagram depicting a further embodiment of the disclosed method, a further aspect of the disclosed method/system shall be described, namely improvement of the clinical algorithm(s) 80 based on feedback indicative of interpretation support accuracy. As illustrated on this figure, in a step 180, feedback is obtained indicative of an accuracy of the interpretation support data O.

According to a first embodiment of the disclosed method/system, the feedback indicative of an accuracy of the interpretation support data O is received from a clinician, health care professional HCP, physician, etc., in particular by way of a user interface. The feedback is indicative of the accuracy of a particular interpretation support data O of a particular patient and/or indicative of the accuracy of a multitude of interpretation support data O related to a plurality of patients - for example an average/ median/ moving average of the accuracy of a risk rating.

In a subsequent step 182, one or more parameters of the clinical algorithm 80 are optimized in response to said feedback in order to improve the accuracy of the interpretation support data O.

According to a further embodiment of the disclosed method/system, the feedback indicative of an accuracy of the interpretation support data O is generated by machine-learning techniques applied over a large amount of interpretation support data O, such as analysis of deviations of the interpretation support data O from statistical data. Additionally or alternatively, interpretation support data O is validated against expected patterns, ratios or plausible values/ rates. For example, for a clinical algorithm 80 which calculates a risk score indicative of the patient to have and/or to acquire a certain medical condition, interpretation support data O comprising such risks scores is analyzed over time and correlated to statistical data indicative of the occurrence of the same medical condition. If a significant deviation is detected, the possible causes of the deviation are investigated and in step 182, parameters of the clinical algorithm 80 are improved/ optimized in order for the clinical algorithm 80 to generate more accurate interpretation support data O in the future.

Embodiments of the disclosed method/system where the virtual analyzer 50 is residing in a remote server (such as cloud environment) are particularly advantageous for analysis of large amounts of interpretation support data O and optimization of the corresponding clinical algorithm(s) 80, since interpretation support data O from several laboratory systems 1 can share the same cloud, enabling a holistic analysis of large amounts of interpretation support data O - usually in anonymized form.

According to the disclosed method/ system, various kinds of clinical algorithms are based on measurement values of different samples and/or of the same sample but different analytical tests and/or the same analytical test but samples obtained at different points in time, etc.

In a first embodiment of the disclosed method/system, a first and second biological sample are obtained from the patient, the second biological sample being obtained from the patient at a different time than the first biological sample. Hence a history of the measurement results - as first input data I - is passed on to the virtual analyzer 50, which then applies a clinical algorithm 80 in order to provide interpretation support data O based thereon.

One example of a clinical algorithm 80 that calculates a risk score (indicative of the patient to have and/or to acquire a certain medical condition) as part of the interpretation support data O based on two subsequent measurements A and B of samples of the same patient obtained at different points in time is the well-known ΔPCT. The ΔPCT clinical algorithm 80 calculates the 28-day mortality risk for patients with severe sepsis or septic shock admitted for intensive care unit ICU care in which Procalcitonin PCT values were measured on Days 0, 1, and 4. In this case, the first input data I comprises the first PCT measurement value A on day 0 or 1 as well as the second PCT measurement value B on day 4. The interpretation support data O then comprises the 28 day Mortality which is also dependent on the second input data II, which comprises an indication whether the patient was discharged from ICU by Day 4. It shall be emphasized that the interpretation support data O is as its name says "support data" and not a diagnosis, which still lies with the health care provided HCP.

In a further embodiment, measurement of samples obtained from a patient at different points in time is used by the virtual analyzer 50 to determine a treatment success rate indicative of the patient responding to a treatment, indications related to the treatment the patient receives/received between the collection of the first respectively second sample being comprised by patient data of the second input data II.

In a further embodiment of the disclosed method/system, the one or more of the plurality of laboratory instruments 10 perform a first analysis of the one or more biological samples of the patient to obtain the first measurement value A and the one or more of the plurality of laboratory instruments 10 performs a second analysis of the one or more biological samples of the patient to obtain the second measurement value A, the first analysis being different from the second analysis. In particular, the first analysis determines presence and/or concentration of a first analyte and the second analysis determines presence and/or concentration of a second analyte, the first analyte being different from the second analyte.

One example of a clinical algorithm 80 that calculates a risk score - as part of the interpretation support data O - based on the first input data I comprising the measurement values indicative of the concentration of different analytes in the same sample of the patient (or aliquots thereof) is the so called ROMA algorithm. The Risk Of Ovarian Malignancy Algorithm ROMA clinical algorithm 80 calculates the risk of epithelial ovarian cancer based on the first measurement value A of CA125 (in pmol/l) and second measurement value B of HE4 (in U/ml). The patient data of the second input data II comprises an indication whether the patient is premenopausal or postmenopausal.

A further feature of embodiments of the disclosed laboratory system 1 is the ability to install/ deploy several clinical algorithms 80.1-80.n on a virtual analyzer 50 in a plug-and-play fashion. When more than one clinical algorithms 80.1-80.n are installed on the same virtual analyzer 50, the workflow engine 52 instructs the algorithm calculator 54 to apply the clinical algorithm 80 and/or the second clinical algorithm 80.2 corresponding to the test order received from the laboratory middleware 30. In other words, the workflow engine 52 of the virtual analyzer 50 selects the right clinical algorithm 80 based on the test order. Hence the workflow engine 52 of the virtual analyzer 50 "manages" the clinical algorithms 80.1-80.n.

According to further embodiments of the disclosed method/system, various clinical algorithms 80.1-80.n are applied by the workflow engine 52 in a reflex-testing manner in that the result of one algorithm triggers the calculation using another algorithm. For example the workflow engine 52 applies the second clinical algorithm 80.2 on the first input data I and second input data II in accordance to an output from applying the clinical algorithm 80.

Further disclosed and proposed is a computer program including computer-executable instructions for performing the method disclosed in one or more of the embodiments enclosed herein when the program is executed on a computer or computer network. Specifically, the computer program may be stored on a computer-readable data carrier. Thus, specifically, one, more than one or even all of the method steps as indicated above may be performed by using a computer or a computer network, preferably by using a computer program.

Further disclosed and proposed is a computer program product having program code means, in order to perform the method disclosed herein in one or more of the embodiments disclosed herein when the program is executed on a computer or computer network. Specifically, the program code means may be stored on a computer-readable data carrier.

Further disclosed and proposed is a data carrier having a data structure stored thereon, which, after loading into a computer or computer network, such as into a working memory or main memory of the computer or computer network, may execute the method according to one or more of the embodiments disclosed herein.

Further disclosed and proposed is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Further disclosed and proposed is a modulated data signal which contains instructions readable by a computer system or computer network, for performing the method according to one or more of the embodiments disclosed herein.

Referring to the computer-implemented aspects of the disclosed method, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Furthermore, hereby disclosed and proposed are:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

It will be understood that many variations could be adopted based on the specific structure hereinbefore described without departing from the scope as defined in the following claims.

**REFERENCE LIST:**

| | |
|---|---|
| laboratory system | 1 |
| laboratory instrument | 10 |
| the laboratory middleware | 30 |
| host system (LIS) | 40 |
| virtual analyzer | 50 |
| workflow engine (of virtual analyzer) | 52 |
| algorithm calculator (of virtual analyzer) | 54 |
| user interface | 56 |
| communication network | 70 |
| clinical algorithm | 80 |
| first measurement value | A |
| second measurement value | B |
| first input data | I |
| second input data | II |
| transmit interpretation support data | O |
| receipt of a test order | step 100 |
| providing a clinical algorithm to the algorithm calculator | step 104 |
| instructing laboratory instrument(s) to analyze sample(s) | step 110 |
| analysis of sample(s) by laboratory instrument(s) | step 120 |
| obtaining measurement values | step 122 |
| receipt of measurement values by laboratory middleware | step 130 |
| data collection and preparation | step 140 |
| processing by the algorithm calculator | step 150 |
| forwarding of interpretation support data | step 160 |
| augmenting measurement values with interpretation support | step 170 |
| obtaining feedback indicative of accuracy of the interpretation support | step 180 |
| optimizing parameters of the clinical algorithm in response to feedback | step 182 |

## Claims

1. A method comprising the steps:
- receiving a test order corresponding to a patient by a laboratory middleware (30);
- one or more laboratory instruments (10) analyzing one or more biological samples of the patient to obtain a first measurement value (A) respectively a second measurement value (B) therefrom in response to an instruction from the laboratory middleware (30) according to the test order;
- the laboratory middleware (30) receiving the first measurement value (A) and the second measurement value (B) obtained by analysis of the one or more biological samples of the patient;
- providing a virtual analyzer (50) comprising a workflow engine (52) and an algorithm calculator (54), the virtual analyzer (50) being distinct from the laboratory middleware (30);
- the workflow engine (52) receiving a first input data (I) comprising the first measurement value (A) and the second measurement value (B) from the laboratory middleware (30);
- the workflow engine (52) receiving from the laboratory middleware (30) a second input data (II) comprising patient data corresponding to the patient;
- providing a clinical algorithm (80) to the algorithm calculator (54), the clinical algorithm (80) defining processing steps to apply on the first input data (I) and second input data (II);
- the workflow engine (52) instructing the algorithm calculator (54) to apply the clinical algorithm (80) on the first input data (I) and second input data (II) in response to the test order received from the laboratory middleware (30);
- the algorithm calculator (54) processing the first input data (I) and second input data (II) using the clinical algorithm (80) and transmit interpretation support data (O) to the workflow engine (52);
- the workflow engine (52) forwarding the interpretation support data (O) to the laboratory middleware (30); and
- the laboratory middleware (30) augmenting the first measurement value (A) and the second measurement value (B) with the interpretation support data (O) received from the virtual analyzer (50).

2. The method according to o claim 1, further comprising the steps:
- obtaining a feedback indicative of an accuracy of the interpretation support data (O);
- optimizing one or more parameters of the clinical algorithm (80) in response to said feedback in order to improve the accuracy of the interpretation support data (O).

3. The method according to claim 1 or 2, further comprising the steps:
- obtaining a first biological sample from the patient;
- obtaining a second biological sample from the patient at a different time than obtaining of the first biological sample;
- the one or more of the plurality of laboratory instruments (10) analyzing the first biological sample of the patient to obtain the first measurement value (A); and
- the one or more of the plurality of laboratory instruments (10) analyzing the second biological sample of the patient to obtain the second measurement value (B).

4. The method according to one of the claims 1 to 3, further comprising the steps:
- the one or more of the plurality of laboratory instruments (10) performing a first analysis of the one or more biological samples of the patient to obtain the first measurement value (A);
- the one or more of the plurality of laboratory instruments (10) performing a second analysis of the one or more biological samples of the patient to obtain the second measurement value (B), the first analysis being different than the second analysis.

5. The method according to claim 4, wherein the first analysis determines presence and/or concentration of a first analyte and the second analysis determines presence and/or concentration of a second analyte, the first analyte being different from the second analyte.

6. The method according to one of the claims 1 to 5, further comprising the step of the laboratory middleware (30) retrieving patient data from a host system (40) such as a hospital information system HIS, an electronic medical record EMR and/or a computerized Physician Order Entry CPOE.

7. The method according to one of the claims 1 to 6, wherein said patient data comprises one or more from the list comprising:
- age of a patient;
- life stage of a patient;
- one or more medical condition(s) of a patient;
- physiological characteristics of a patient, such as weight, height, gender, etc.
- a list of past and/or current drug(s) administered to the patient and respective dosage(s);
- past and/or current treatments of the patient;
- patient demographics.

8. The method according to one of the claims 1 to 7, wherein processing of the first input data (I) and second input data (II) using the clinical algorithm (80) comprises determination of a risk score indicative of the patient to have and/or to acquire a certain medical condition, the interpretation support data (O) comprising said risk score.

9. The method according to one of the claims 3 to 8, wherein processing of the first input data (I) and second input data (II) using the clinical algorithm (80) comprises determination of a treatment success rate indicative of the patient responding to a treatment based on the patient data and based on measurement of the first biological sample and of the second biological sample obtained at a different time than obtaining of the first biological sample, the interpretation support data (O) comprising said treatment success rate.

10. The method according to one of the preceding claims, further comprising the steps:
- providing a second clinical algorithm (80.2) to the algorithm calculator (54), the second clinical algorithm (80.2) defining processing steps to apply on the first input data (I) and second input data (II), the clinical algorithm (80) being different from the second clinical algorithm (80.2);
- the workflow engine (52) instructing the algorithm calculator (54) to apply the clinical algorithm (80) and/or the second clinical algorithm (80.2) corresponding to the test order received from the laboratory middleware (30).

11. The method according to claim 10, further comprising the step: the workflow engine (52) applying the second clinical algorithm (80.2) on the first input data (I) and second input data (II) in accordance to an output from applying the clinical algorithm (80).

12. A laboratory system (1) for processing biological samples comprising:
- a plurality of laboratory instruments (10) for analyzing biological samples, each laboratory instrument (10) providing a measurement value obtained by analysis of a biological sample;
- a laboratory middleware (30) communicatively connected to the plurality of laboratory instruments (10) and
- a virtual analyzer (50) comprising a workflow engine (52) and an algorithm calculator (54), the algorithm calculator (54) comprising one or more clinical algorithms (80),
wherein the laboratory system (1) is configured to perform the steps of any one of the methods according to claims 1 through 11.

13. A computer-readable medium storing instructions thereon which when executed by a computer system controls the computer system to perform the steps of any one of the methods according to claims 1 through 11.

14. A web-based system comprising:
- a user interface (56) for receiving first input data (I) indicative of the first measurement value (A) and the second measurement value (B) obtained by analysis of the one or more biological samples of the patient and for receiving second input data (II) comprising patient data corresponding to the patient;
- a virtual analyzer (50) comprising a workflow engine (52) and an algorithm calculator (54) comprising one or more clinical algorithm(s) (80);
wherein the web-based system is configured to perform the steps of any one of the methods according to claims 1 through 11.

15. The web-based system according to claim 14, wherein the virtual analyzer (50) resides on a remote server, in particular a cloud-based remote server.
